# FASCICULE DE BREVET EUROPEEN

(11) **EP 0 141 686 B1**
(45) Date de publication et mention de la délivrance du brevet: **09.09.1987**
(21) Numéro de dépôt: 84401595.8
(22) Date de dépôt: 30.07.1984
(51) Int. Cl.: C07D 403/04, C07D 405/06, A61K 31/415, A61K 31/40, A61K 31/36

(54) **Dérivés de l'indole, leur préparation et leur application en thérapeutique**
Indol-Derivate, Verfahren zu deren Herstellung und deren therapeutische Anwendung
Indole derivatives, their preparation and their therapeutical application

(30) Priorité: 11.08.1983 FR 8313198; 17.10.1983 FR 8316473; 15.11.1983 FR 8318121; 09.02.1984 FR 8401998
(43) Date de publication de la demande: 15.05.1985
(73) Titulaire: SYNTHELABO, 92350 Le Plessis Robinson (FR)
(72) Inventeur: Bigg, Dennis, F-78350 Jouy en Josas (FR); Menin, Jacques, F-94400 Vitry sur Seine (FR)
(74) Mandataire: Thouret-Lemaitre, Elisabeth

## Description

La présente invention concerne des dérivés de l'indole, leur préparation et leur application en thérapeutique.

Des dérivés d'imidazole ont déjà été décrits dans la littérature, par exemple dans la demande de brevet européen 00 77 024 ; ces composés diffèrent cependant struçturalement (ils ne comportent pas de radical indolyle) et par leurs propriétés thérapeutiques (antiinflammatoires) des composés de l'invention (α₂-antagonistes).

Les composés de l'invention répondent à la formule (I) 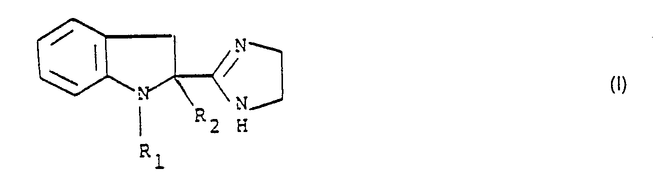 dans laquelle
R₁ est un atome d'hydrogène, un radical (C₁₋₆)alkyle droit ou ramifié, un radical (C₃₋₆)cycloalkyl-(C₁₋₄)alkyle, le radical allyle, le radical naphtylméthyle, le radical phénéthyle ou un radical benzyle pouvant porter un ou plusieurs substituants choisis parmi les atomes d'halogène et les radicaux méthyle, méthoxy et méthylènedioxy, et
R₂ est H ou un radical (C₁₋₄) alkyle ou allyle.

Les composés de l'invention peuvent exister, sous la forme de racémates ou d'énantiomères qui font partie de l'invention.

Les sels pharmaceutiquement acceptables des composés (I) font partie de l'invention.

Les composés de l'invention pour lesquels R₁ est différent de H peuvent être préparés selon le schéma réactionnel suivant : 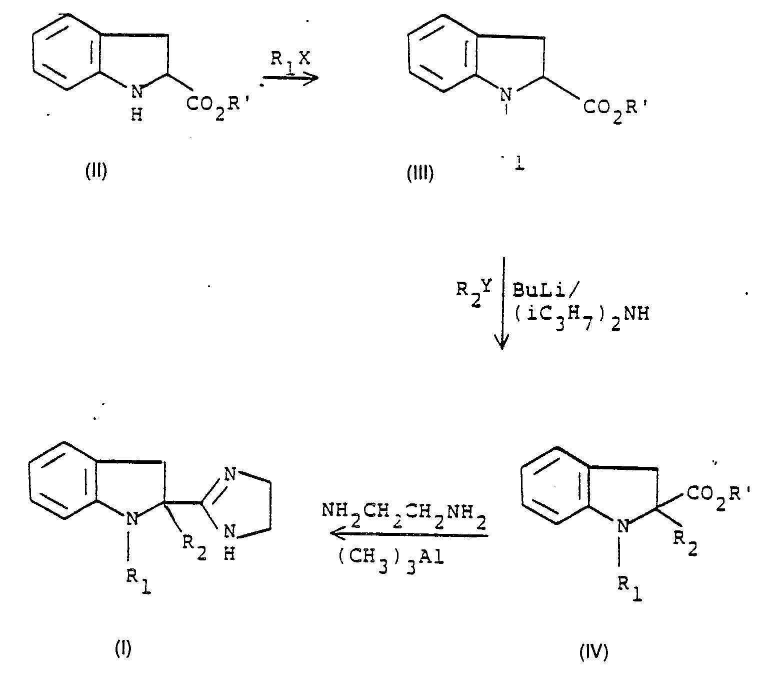 dans lequel R' est un radical (C₁₋₄) alkyle, en particulier éthyle, et X et Y sont des groupes labiles, en particulier des atomes d'iode ou de brome.

Les composés de l'invention pour lesquels R, et H peuvent être préparés selon l'un des deux schémas réactionnels. suivants :
Schéma 1 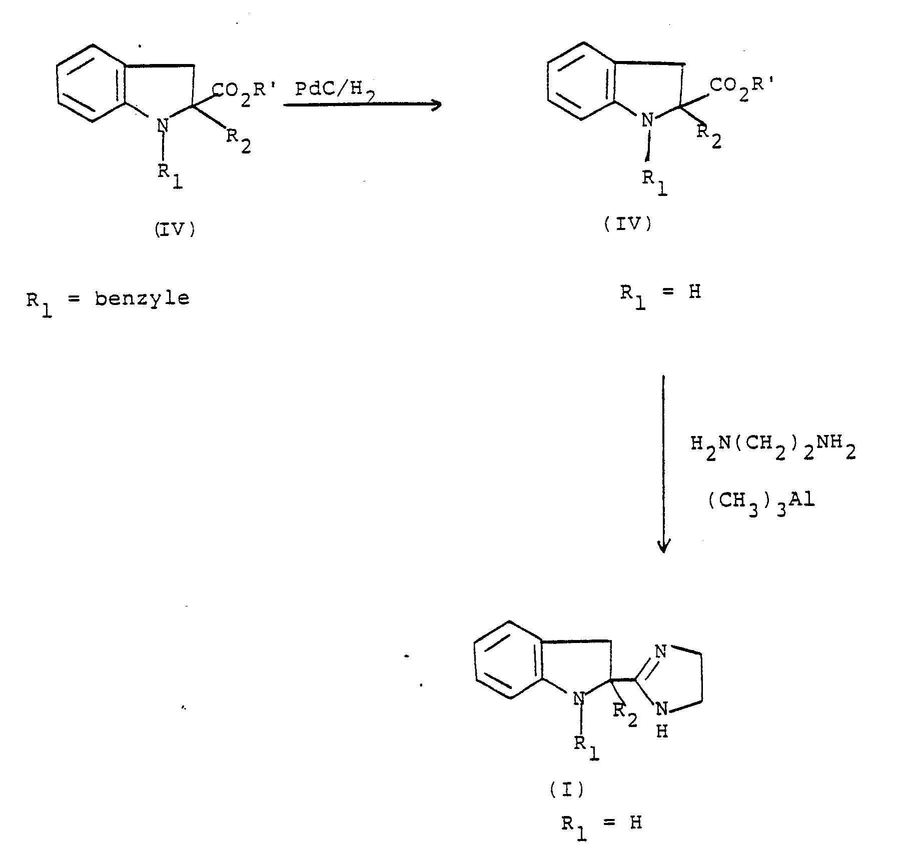
Schéma 2

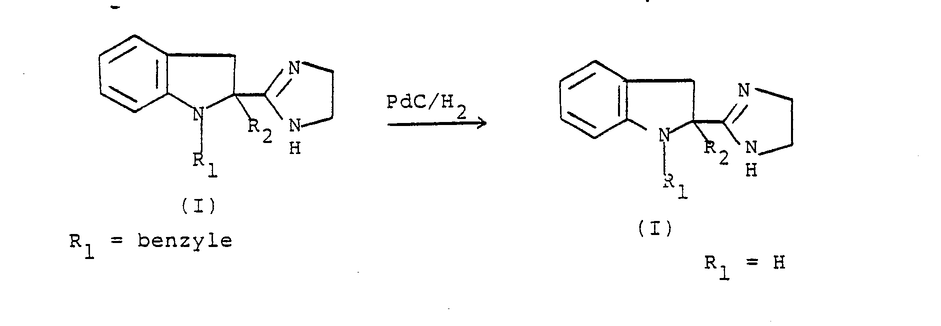

L'ester éthylique de départ (formule II) est préparé selon la méthode décrite par E. J. Corey et al. J. Am. Chem. Soc., 92, 2476 (1970).

La réaction entre l'ester (II) de départ, par exemple le dihydro-2,3 1 H-indolecarboxylate-2 d'éthyle, et le composé R₁X est effectuée dans un solvant tel que l'acétone, la méthyléthylcétone ou le diméthylforma- mide, à la température ambiante ou à température plus élevée, en présence d'une base, telle que le carbonate de potassium. La réaction peut-être catalysée par exemple par addition d'iodure de sodium.

L'alkylation ou l'alcénylation éventuelle de l'ester (III) ainsi obtenu est effectuée par action d'un halogénure R₂Y sur le dérivé lithié préparé in situ au moyen de diisopropylamidure de lithium (butyllithium + diisopropytamine).

La formation de l'imidazoline à partir de. l'ester (III) ou de l'ester (IV) est effectuée par l'éthylènedia- mine en présence de triméthylaluminium.

La débenzylation du composé (IV) ou du composé (I) dans lequel R₁ est benzyle pour obtenir les composés (I) dans lesquels R, est H, est effectuée à l'aide d'hydrogène sous pression en présence de palladium sur charbon.

Les exemples suivants et le tableau illustrent l'invention. Les analyses et les spectres IR et RMN confirment la structure des composés obtenus selon l'invention.

### Exemple 1 (dihydro-4,5 1H-imidazolyl-2)-2 méthyl-1 dihydro-2,3 1H-indole

La réaction est effectuée sous azote ou argon.

On ajoute 2,28 ml (0,034 mole) d'éthylènediamine en solution dans 6 ml de toluène à une solution froide de triméthylaluminium (14,2 ml à 25 % dans l'hexane ; 0,034 mole) dans 22 ml de toluène.

On chauffe le mélange réactionnel à 50-60 °C et ajoute 4,4 g (0,021 mole) de dihydro-2,3 ethoxycar- bonyl-2 méthyl-1 1H-indole dans 6,3 ml de toluène.

On chauffe le mélange réactionnel à la température du reflux et élimine environ 30 ml de solvant à l'aide d'un appareil Dean-Stark, on maintient la température du reflux pendant 12 heures, on refroidit le mélange réactionnel et ajoute 14 ml d'eau, on filtre et rince le précipité avec de l'acétate d'éthyle.

On réunit les phases organiques, les lave une fois avec de l'eau saturée de chlorure de sodium. On sèche la phase organique sur MgS0₄, filtre et évapore.

On dissout la base obtenue dans 50 ml d'éthanol et la traite avec une solution d'acide furamique dans de l'éthanol. On fait recristalliser le sel obtenu dans l'éthanol.
F = 178-180 °C

### Exemple 2 (dihydro-4,5 1H-imidazolyl-2)-2 6enzyl-1 dihydro-2,3 1H-indole

La réaction est effectuée sous azote ou argon.

On ajoute 1,8 ml (0,027 mole) d'éthylènediamine en solution dans 5 ml de toluène à une solution froide de triméthylaluminium (11,4 ml à 25 % dans de l'hexane : 0,027 mole) dans 18 ml de toluène.

On chauffe le mélange réactionnel à 50-60 °C et ajoute 4,8 g (0,017 mole) de dihydro-2,3 -éthoxycarbonyl-2 benzyl-1 1H-indole dans du toluène.

On chauffe le mélange réactionnel à la température du reflux et élimine environ 15 ml de solvant à l'aide d'un appareil Dean-Stark, on maintient la température du reflux pendant 2 h 30, on refroidit le mélange réactionnel et ajoute 11 ml d'eau, on filtre et rince le précipité avec de l'acétate d'éthyle.

On réunit les phases organiques, les lave une fois avec de l'eau saturée de chlorure de sodium. On sèche la phase organique sur MgS0₄, filtre et évapore.

On dissout la base obtenue dans 50 ml d'éthanol et la traite avec une solution d'acide fumarique dans de l'éthanol.

On fait recristalliser le sel obtenu dans de l'éthanol.
F = 172-173, 5°C.

### Exemple 3 (dihydro-4,5 1 H-imidazolyl-2)-2 (méthoxy-4 benzyl)-1 dihydro-2.3 1H-indole

1. Sous argon, on introduit 5,16 g (0,027 mole) de dihydro-2,3 1 H-indolecarboxylate-2 d'éthyle et 6 g (0,043 mole) de K₂CO₃ dans 50 ml de DMF.

Puis on ajoute 4,65 g (0,0297 mole) de chlorure de méthoxy-4 benzyle et 4 g (0.,027 mole) de Nal.

On agite le mélange réactionnel pendant 4 heures à la température ambiante, puis on le verse dans de l'eau glacée et extrait à l'éther. On lave la solution éthérée à l'eau, puis avec du NaHS0₃ dilué puis avec de l'eau. On sèche, filtre et concentre. On obtient le (méthoxy-4 benzyl)-1 dihydro-2,3 1H-indolecarboxylate-2 d'éthyle, brut, que l'on utilise tel quel dans la deuxième étape.

2. La réaction est effectuée sous argon. On ajoute 2,55 ml (0,038 mole) d'éthylènediamine en solution dans 10 ml de toluène à une solution froide de triméthylaluminium (16,15 ml à 25 % dans l'hexane ; 0,038 mole) dans 28 ml' de toluène.

On chauffe le mélange réactionnel à 50-60 °C et ajoute 7,5 g (0,024 mole) de l'ester obtenu précédemment en solution dans 27 ml de toluène.

On chauffe le mélange réactionnel à la température du reflux et élimine environ 20 ml de solvant à l'aide d'un appareil Dean-Stark ; on maintient la température du reflux pendant 12 heures ; on refroidit le mélange réactionnel et ajoute 16 ml d'eau, on filtre et rince le précipité avec du dichlorométhane.

On réunit les phases organiques, les lave une fois avec de l'eau. On sèche la phase organique sur MgS0₄, filtre et évapore. On obtient le produit sous forme d'huile que l'on dissout dans 150 ml d'éthanol et que l'on traite avec une solution d'acide fumarique (2.1 g) dans de l'éthanol (100 ml).

Le produit cristallise lentement. On le filtre et le fait cristalliser dans de l'éthanol.
F = 154-156 °C.

### Exemple 4 (dihydro-4,5 1H-imidazolyl-2)-2 n-butyl-1 dihydro-2,3 1H-indole

1. Sous argon, on introduit dans un ballon 5,16 g (0,027 mole) de dihydro-2,3 1H-indolecarboxylate-2 d'éthyle et 6,0 g (0,043 mole) de K₂CO₃ dans 50 ml de DMF.

Puis on ajoute 22,0 g (0,12 mole) de iodobutane et on chauffe le mélange à 60 °C pendant 9 heures tout en agitant. On le verse ensuite sur un mélange d'eau et de glace, on l'extrait à l'éther, on lave, sèche et concentre la phase organique. On obtient une huile jaune qui, purifiée par chromatographie puis par distillation, présente un point d'ébullition de 120°C sous une pression de 0,7 Pa (0,005 mmHg).

2. A une solution de 1,55 g (0,0214 mole) de triméthylaluminium dans 15 ml de toluène, à 0-5 °C, on ajoute une solution de 1,33 g (0,0221 mole) d'éthylènediamine dans 5 ml de toluène. On chauffe le mélange à 50 °C, et on ajoute 3,4 g (0,014 mole) de n-butyl-1 dihydro-2,3 1H. indolecarboxylate d'éthyle en solution dans 20 ml de toluène et on porte le tout au reflux pendant 24 heures.

Ensuite on hydrolyse le mélange avec 20 ml d'eau, on sépare le précipité par filtration et on le rince à l'acétate d'éthyle. On réunit les phases organiques, on les lave à l'eau, on les sèche et on les concentre. On obtient une huile qui cristallise dans l'éther de pétrole en formant un solide blanc.

On reprend ce solide blanc dans de l'éthanol et on le traite avec un équivalent d'acide fumarique. On concentre le mélange, on le triture dans l'acétone, et on recristallise le solide obtenu dans un mélange acétonelalcool isopropylique.
F = 111,5-113°C.

### Exemple 5 diméthyl-1,2 (dihydro-4,5 1H-imidazolyl-2)-2 dihydro-2.3 1H-indole

1. Sous argon et à - 78 °C, on introduit dans un ballon 5 ml (0,036 mole) de diisopropylamine dans 30 ml de tétrahydrofuranne et 22,5 ml (0.036 mole) de butyllithium à 1,6 M dans l'hexane.

On agite une heure à -78 °C, puis on introduit 6,2 g (0,030 mole) de méthyl-1 dihydro-2,3 1H-indolecarboxylate-2 d'éthyle dissous dans 20 ml de tétrahydrofuranne, et on continue d'agiter pendant une heure à -78 °C. Enfin on ajoute 21,3 g, soit 9,3 ml (0,150 mole) de iodométhane, on agite encore une heure à -78 °C et on laisse revenir le mélange à la température ambiante.

On le verse alors sur un mélange d'eau et de glace, on l'extrait à l'éther, lave la phase organique à l'eau, la sèche et la concentre. On obtient/une huile orange qu'on purifie par chromatographie sur silice en éluant avec un mélange 98/2 de cyclohexane/acétate d'éthyle.

Après distillation l'huile obtenue à un point d'ébullition de 110-115 °C sous 0,67 Pa (0,005 mmHg).

2. Sous argon, à une solution de 1,34 g (0,0185 mmole) de triméthylaluminium (7,75 ml d'une solution à 25 % dans l'hexane) dans 15 ml de toluène on additionne, entre 0 et 5°C. au goutte à goutte et en l'espace de 30 minutes, une solution de 1,14 g, soit 1,3 ml (0,019 mole) d'éthylènediamine dans 5 ml de toluène, en maintenant la température entre 0 et 5 °C. Puis on chauffe le mélange à 50 °C et on ajoute, goutte à goutte, 2,6 g (0,012 mole) de l'huile précédemment obtenue en solution dans 20 ml de toluène. En fin d'addition, on chauffe à 80-90 °C, on distille l'hexane et on porte au reflux pendant 18 heures en suivant la réaction par chromatographie sur couche mince.

Il reste finalement une solution limpide jaune, que l'on hydrolyse par 20 ml d'eau, en refroidissant sur bain de glace. On filtre la solution blanchâtre obtenue, on l'extrait au chlorure de méthylène, on lave, sèche et concentre l'extrait. On obtient ainsi une huile orange que l'on reprend par de l'éther, et on verse cette solution dans une solution éthérée d'acide benzoïque. Un solide blanc précipite, on le sépare et on le recristallise dans de l'acétate d'éthyle. le benzoate fond à 118,5-120 °C.

### Exemple 6 (dihydro-4,5 1H-imidazolyl-2)-2 n-propyl-2 dihydro-2,3 1H indole.

Ce composé a été préparé selon les schémas réactionnels 1 et 2.

### 1. Schéma 1

1.1 Dans un appareil de Parr de 500 ml, on introduit 9,75 g 0,03 mole) d'éthoxycarbonyl-2 n-propyl-2 benzyl-1 dihydro-2,3 1 H-indole dans 100 ml d'acide acétique et 0,5 g de palladium sur charbon à 10 %. On introduit de l'hydrogène sous une pression de 0,4 MPa et on agite à la température ambiante pendant 8 heures. On filtre le mélange réactionnel, le concentre, le reprend à l'éther, lave avec une solution aqueuse de NaHC0₃ puis à l'eau jusqu'à pH 6-7. On sèche sur MgS0₄, filtre, concentre le mélange réactionnel et le distille au four à boules.

On obtient l'éthoxycarbonyl-2 n-propyl-2 dihydro-2,3 1H-indole sous forme d'huile jaune.

1.2. Dans un ballon de Keller de 250 ml muni d'une agitation magnétique, d'un réfrigérant, d'une arrivée d'argon et d'une ampoule à introduction, on introduit successivement 70 ml de toluène et 34,4 ml (0,082 mole) d'une solution de triméthylaluminium à 25 % dans de l'hexane.

On refroidit le mélange réactionnel par un bain glacé et ajoute une solution de 5,5 ml (0,082 mole) d'éthylènediamine dans 25 ml de toluène.

On chauffe le mélange vers 50-60 °C et ajoute une solution de 6 g (0,0257 mole) du composé précédemment obtenu sous 1.1. dans 30 ml de toluène.

On chauffe le mélange à la température du reflux pendant 2 h. On refroidit vers environ -10°C et hydrolyse par 35 ml d'eau. On filtre, rince à l'acétate d'éthyle, puis au CH₂CI₂. Les phases organiques sont réunies puis lavées à l'eau deux fois. On sèche sur MgS0₄, filtre et concentre.

L'huile obtenue est reprise dans 100 ml d'ortho-dichlorobenzène. On y ajoute 1 pointe de spatule d'acide p-toluènesulfonique et sous atmosphère d'argon on chauffe à la température de reflux en surmontant le ballon d'un Dean-Stark pendant 18 h.

On reprend le mélange dans du chlorure de méthylène, lave à l'eau 2 fois, sèche sur MgS0₄, filtre et concentre. Après un traitement au noir animal, on transforme la base obtenue en fumarate en faisant réagir 2,7 g (0,0231 mole) d'acide fumarique dissous dans 135 ml d'éthanol avec 5,8 g (0,0257 mole) de base dissoute dans 50 ml d'éthanol.

On concentre, reprend dans l'acétone. Le produit cristallise. On le filtre et le fait recristalliser dans de l'isopropanol puis dans de l'éthanol. Le produit fond à 167-169 °C.

### 2. Schéma 2

On introduit 4,4 g (0,0137 mole) de (dihydro-4,5 1H-imidazolyl-2)-2 n-propyl-2 benzyl-1 dihydro-2,3 1Ji- indole en solution dans 100 ml d'acide acétique dans un appareil de Parr de 500 ml. On ajoute 0,44 g de palladium sur charbon à 10 % puis on introduit de l'hydrogène sous une pression, de 0,4 MPa. On chauffe le mélange 8 h à 80-90 °C.

On filtre, concentre et reprend à l'eau. On alcalinise le mélange avec de la lessive de soude et extrait au chlorure de méthylène. La phase organique est lavée à l'eau 3 fois, séchée sur MgS0₄, filtrée et concentrée.

On transforme la base en fumarate en faisant réagir 3,2 g (0.0137 mole) de base dissoute dans 50 ml d'éthanol avec 1,54 g (0,0132 mole) d'acide fumarique dissous dans 75 ml d'éthanol. On concentre, reprend dans de l'isopropanol, filtre et fait recristalliser le composé dans de l'éthanol.
F = 167-169 °C.

Dans le tableau suivant sont représentés les composés de l'invention préparés à titre d'exemples.
(Voir tableaux page 7 et s.)

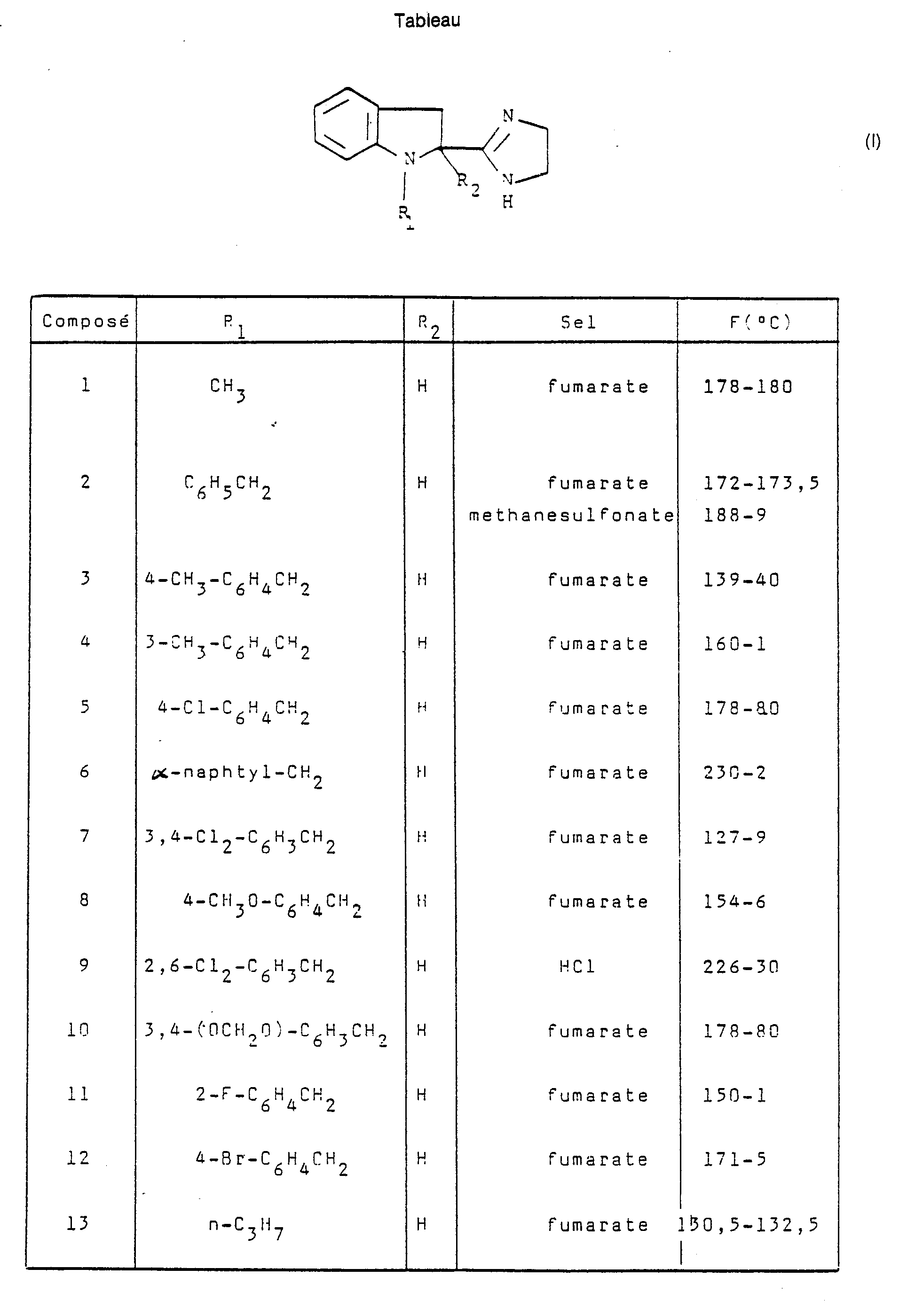

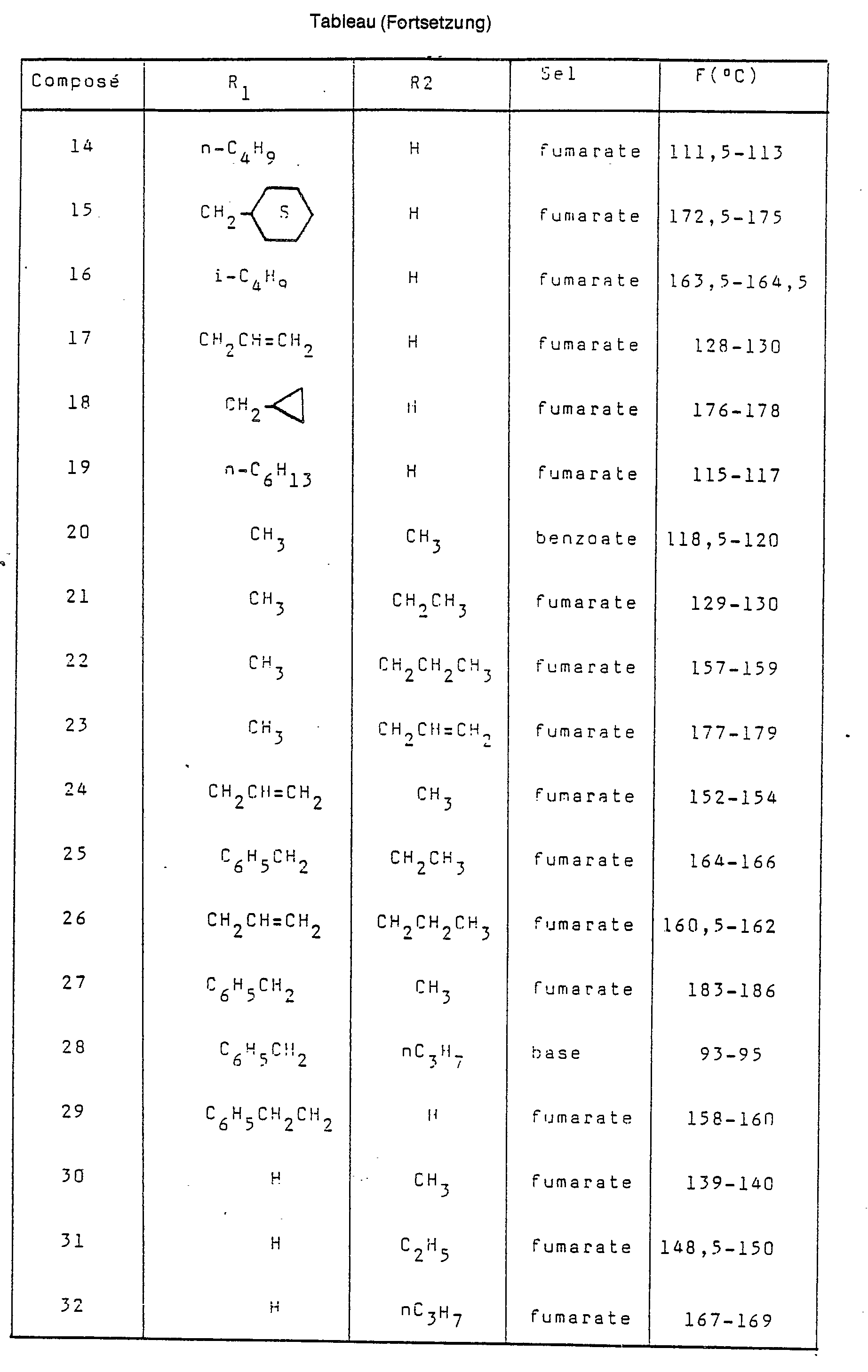

Les composés de l'invention ont été soumis à des essais pharmacologiques qui ont montré leur intérêt en tant que α₂-antagonistes.

A cet effet les composés ont été étudiés dans le test de potentialité et de sélectivité des antagonistes à l'égard des récepteurs α₂ in vitro.

La détermination de la valeur pA₂ à l'égard des effets inhibiteurs de la clonidine, α₂-agoniste bien connu, a eu lieu sur le vas deferens du rat stimulé à une fréquence de 0,1 Hz en présence de 30 nM de prazosine et de 1 µm de cocaïne, selon la méthode décrite par G. M. Drew (European Journal of Pharmacology, 42, (1977) 123-130).

Les pA₂ des composés de l'invention sont compris entre 6 et 10.

Les composés de l'invention sont des α₂-antagonistes puissants qui peuvent être utilisés pour le traitement de la dépression (soit seul, soit en association avec un produit qui inhibe les mécanismes de captation neuronale), le traitement de l'hypotension, le traitement de l'ileum paralytique post-opératoire, le traitement de l'asthme et de l'obésité.

Les compositions pharmaceutiques peuvent être sous forme appropriée pour l'administration par voie orale, rectale ou parentérale ; par exemple sous la forme de capsules, comprimés, granulés, gélules ou solutés liquides, sirops ou suspensions buvables, et contenir les excipients appropriés.

La posologie quotidienne peut aller de 0,1 à 10 mg/kg p. o.

## Revendications (Revendications pour l'(les) Etat(s) contractant(s) suivant(s) : FR, DE, GB, BE, IT, LU, NL, SE, CH et LI)

1. Dérivés de l'indole, sous la forme de racémates ou d'isomères optiquement actifs, répondant à la formule (I) 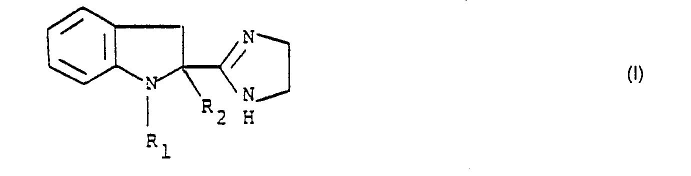 dans laquelle
R, est un atome d'hydrogène, un radical (C₁₋₆)alkyle droit ou ramifié, un radical (C₃₋₆)cycloalkyl-(C₁₋₄)alkyle, le radical allyle, le radical naphtylméthyle, le radical phénéthyle ou un radical benzyle pouvant porter un ou plusieurs substituants choisis parmi les atomes d'halogène et les radicaux méthyle, méthoxy et méthylènedioxy, et
R₂ est H ou un radical (C₁₋₄)alkyle ou allyle ; ainsi que leurs sels d'addition aux acides pharmaceutiquement acceptables.

2. Composés selon la revendication 1, dans lesquels R, est soit un radical benzyle éventuellement substitué par un atome d'halogène ou un radical méthyle ou méthylènedioxy, soit le radical allyle, soit un radical (C₁₋₄)alkyle, et R₂ est H.

3. Procédé de préparation des composés (I) selon la revendication 1, dans lesquels R₁ est différent de H, procédé caractérisé en ce que l'on fait réagir un ester de formule (II) 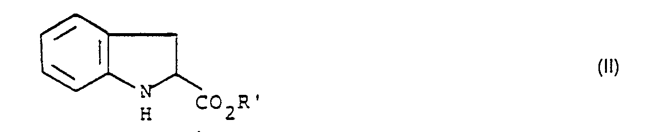 avec un composé R, X pour obtenir le composé (III) 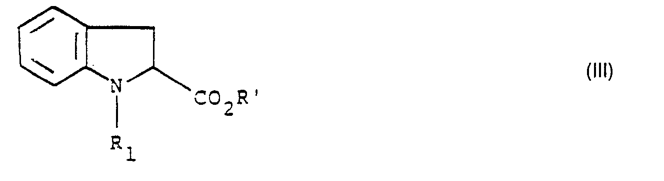 que l'on alkyle ou allyle éventuellement par réaction avec un halogénure R₂ Y en présence de butyllithium et de diisopropylamine pour obtenir le composé (IV) 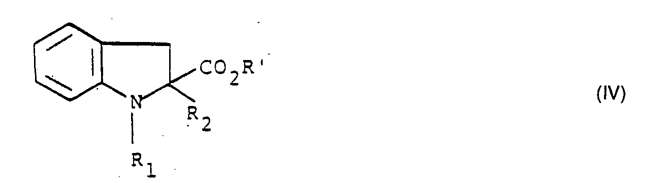 et on transforme en composé (I) le composé (III) ou le composé (IV), en le faisant réagir avec de l'éthylènediamine en présence de triméthylaluminium ; X et Y étant des groupes labiles, en particulier des atomes d'iode ou de brome, R' étant un radical (C₁₋₄)alkyle, et R₁ et R₂ ayant les significations données dans la revendication 1.

4. Procédé.de préparation des composés (I) selon la revendication 1, dans lesquels R, est H, procédé caractérisé en ce que
soit l'on débenzyle un composé (IV) dans lequel R₁ est le radical benzyle, puis on transforme le composé obtenu (IV) dans lequel R₁ est H en composé (I) dans lequel R₁ est H en le faisant réagir avec de l'éthylènediamine en présence de triméthylaluminium ;
soit l'on débenzyle un composé (I) dans lequel R₁ est de radical benzyle.

5. Médicament caractérisé en ce qu'il contient un composé tel que spécifié dans l'une quelconque des revendications 1 et 2.

6. Composition pharmaceutique caractérisée en ce qu'elle contient un composé de l'une quelconque des revendications 1 et 2, en association avec tout excipient approprié.

## Revendications (Revendications pour l'(les) Etat(s) contractant(s) suivant(s) : AT)

Procédé de préparation de dérivés de l'indole, sous la forme de racémates ou d'isomères optiquement actifs, répondant à la formule (I) 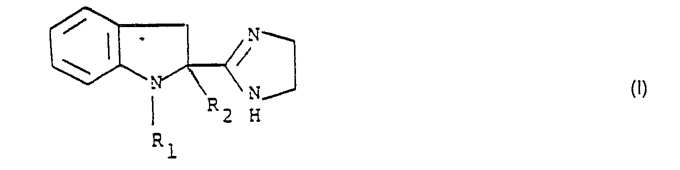 dans laquelle
R₁ est un atome d'hydrogène, un radical (C₁₋₆)alkyle droit ou ramifié, un radical (C₃₋₆)cycloalkyl-(C₁₋₄)alkyle, le radical allyle, le radical naphtylméthyle, le radical phénéthyle ou un radical benzyle pouvant porter un ou plusieurs substituants choisis parmi les atomes d'halogène et les radicaux méthyle, méthoxy et méthylènedioxy, et
R₂ est H ou un radical (C₁₋₄)alkyle ou allyle,
procédé caractérisé en ce que
soit on prépare les composés dans lesquels R₁ est différent de H, et alors on fait réagir un ester de formule (II) 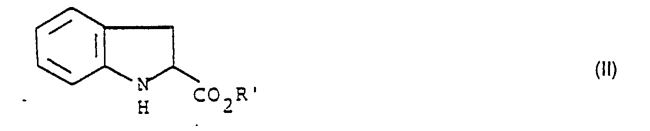 avec un composé R₁ X pour obtenir le composé (III) 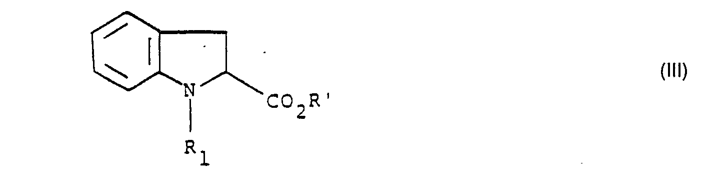 que l'on alkyle ou allyle éventuellement par réaction avec un halogénure R₂ Y en présence de butyllithium et de diisopropylamine pour obtenir le composé (IV) 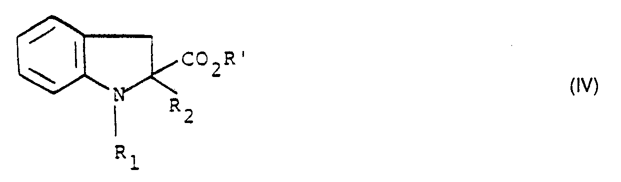 et on transforme en composé (I) le composé (lll) ou le composé (IV), en le faisant réagir avec de l'éthylènediamine en présence de triméthylaluminium ; X et Y étant des groupes labiles, en particulier des atomes d'iode ou de brome, R' étant un radical (C₁₋₄)alkyle,
soit on prépare les composés dans lesquels R₁ est H, et alors
on débenzyle un composé (IV) dans lequel R₁ est le radical benzyle, puis on transforme le composé obtenu (IV) dans lequel R₁ est H en composé (I) dans lequel R₁ est H en le faisant réagir avec de l'éthylènediamine en présence de triméthylaluminium ;
ou
on débenzyle un composé (I) dans lequel R₁ est le radical benzyle.

## Claims (Claims for the following Contracting State(s) : BE, CH, DE, FR, GB, IT, LI, LU, NL, SE)

1. Indole derivatives, in the form of racemates or optically active isomers, corresponding to the formula (I) 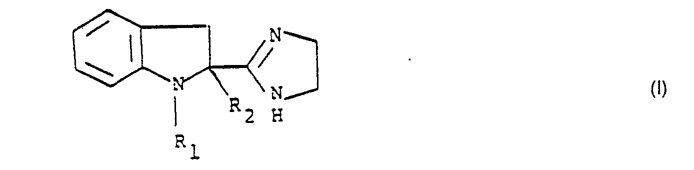 in which
R₁ is a hydrogen atom, a linear or branched (C₁₋₆)alkyl radical, a (C₃₋₆)cycloalkyl-(C₁₋₄)alkyl radical, the allyl radical, the naphthylmethyl radical, the phenethyl radical or a benzyl radical which may carry one or more substituents selected from halogen atoms and methyl, methoxyl and methylenedioxy radicals and
R₂ is H or a (C₁₋₄)alkyl or allyl radical, and their addition salts with pharmaceutically acceptable acids.

2. Compounds according to claim 1, in which R, is either a benzyl radical optionally substituted by a halogen atom or a methyl or methylenedioxy radical, or the allyl radical, or a (C₁₋₄) alkyl radical, and R₂ is H.

3. Process for the preparation of compounds according to claim 1, in which R₁ is other than H, a process in which an ester of the formula (II) 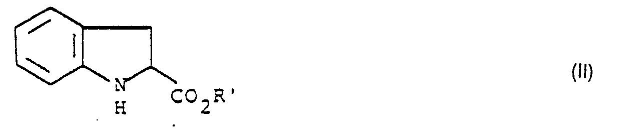 is reacted with a compound R₁X to obtain the compound (III) 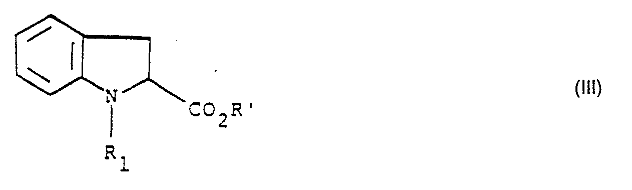 which is optinally alkylated or allylated by reaction with a halide R₂Y in the presence of butyllithium and diisopropylamine to obtain the compound (IV) 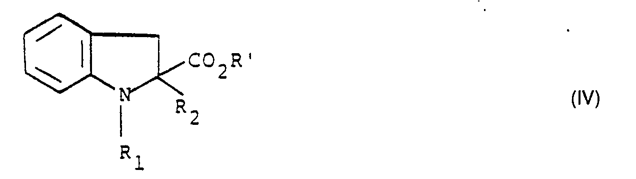 and the compound (III) or the compound (IV) is converted into compound (I) by reacting it with ethylenediamine in the presence of trimethylaluminium ; X and Y being labile groups, in particular iodine atoms or bromine atoms, R' is a (C₁₋₄)alkyl radical, and R₁ and R₂ have the meanings given in claim 1.

4. Process for the preparation of compounds (I) according to claim 1, in which R₁ is H, process in which
either a compound (IV) is debenzylated, in which R₁ is the benzyl radical, and then the compound (IV) obtained in which R₁ is H is converted to a compound (I) in which R₁ is H by being made to react with ethylenediamine in the presence of trimethylaluminium ;
or a compound (I) in which R₁ is the benzyl radical is debenzylated.

5. A medicament which contains a compound such as specified in either of claims 1 and 2.

6. A pharmaceutical composition which contains a compound of either of claims 1 and 2, associated with any suitable excipient.

## Claims (Claims for the following Contracting State(s) : AT)

A process for the preparation of indole derivatives, in the form of racemates or optically active isomers, corresponding to the formula (I) 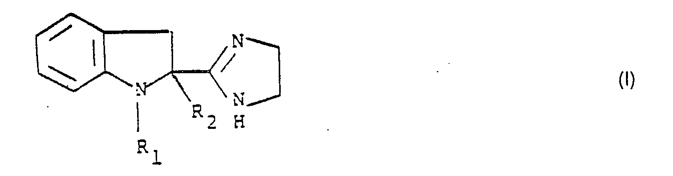 in which
R₁ is a hydrogen atom, a linear or branched (C₁₋₆)alkyl radical, a(C₃₋₆)cycloalkyl-(C₁₋₄)alkyl radical, the allyl radical, the naphthylmethyl radical, the phenethyl radical or a benzyl radical which may carry one or more substituents selected from halogen atoms and methyl, methoxyl and methylenedioxy radicals and
R₂ is H or a (C₁₋₄) alkyl or allyl radical,
process characterized in that
either there are prepared the compounds wherein R, is other than H, in which case an ester of the formula (ll) 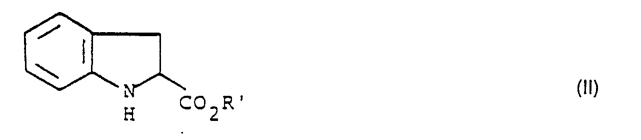 is reacted with a compound R,X to obtain the compound (III) 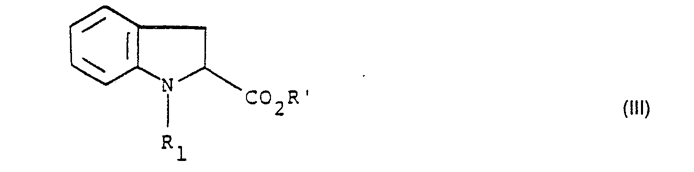 which is optionally alkylated or allylated by reaction with a halide R_{Z}Y in the presence of butyllithium and diisopropylamine to obtain the compound (IV) 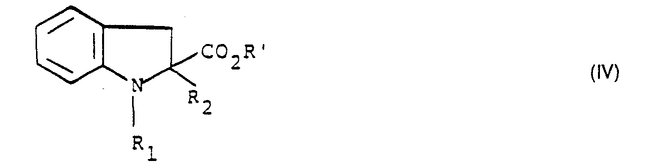 and the compound (III) or the compound (IV) is converted into compound (I) by reacting it with ethylenediamine in the presence of trimethylaluminium ; X and Y being labile groups, in particular iodine atoms or bromine atoms, R' is a (C₁₋₄)alkyl radical, and R₁ and R₂ have the meanings given above,
or there are prepared the compounds wherein R₁ is H, in which case
either a compound (IV) is debenzylated, in which R₁ is the benzyl radical, and then the compound (IV) obtained in which R₁ is H is converted to a compound (I) in which R₁ is H by being made to react with ethylenediamine in the presence of trimethylaluminium ;
or a compound (I) in which R₁ is the benzyl radical is debenzylated.

## Patentansprüche (Patentansprüche für folgende(n) Vertragsstaat(en) : BE, CH, DE, FR, GB, IT, LI, LU, NL, SE)

1. Indol-Derivate, in Form ihrer Racemate oder Enantiomeren, der Formel (I) 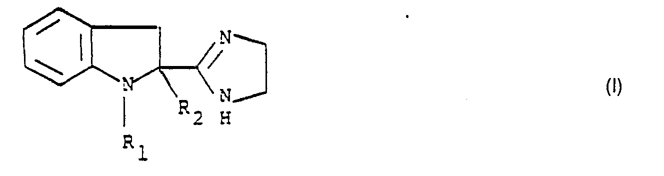 in welcher
R, ein Wasserstoffatom, einen geradkettigen oder verzweigten (C₁₋₆)Alkylrest, einen (C₃₋₆) Cycloalkyl-(C₁₋₄)Alkylrest, den Allylrest, den Naphtylmethylrest, den Phenethylrest oder einen Benzylrest, der ein oder wehrere Halogenatome, Methylgruppen, Methoxygruppen oder Methylenedioxygruppen tragen kann, bedeutet,
R₂ einen Wasserstoffatom, einen (C₁₋₄)Alkylrest oder einen Allylrest bedeutet, sowie deren Additionssalze mit pharmazeutisch annehmbaren Saüren.

2. Derivate nach Anspruch 1, dadurch gekennzeichnet, dass R, entweder einen Benzylrest, der ein Halogenatom oder eine Methylgruppe oder Methoxygruppe tragen kann, oder einen Allylrest oder einen (C₁₋₄)Alkylrest bedeutet und R₂ ein Wasserstoffatom ist.

3. Verfahren zur Herstellung der Verbindungen nach Anspruch 1, in welcher R₁ kein Wasserstoffatom ist, dadurch gekennzeichnet, dass man ein Ester der Formel (11) 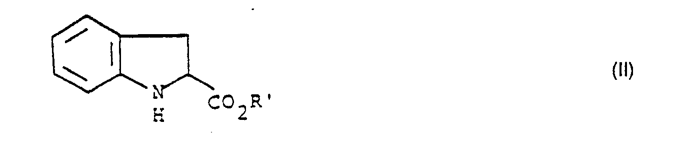 mit einer Verbindung R,X reagiert, um die Verbindung (lll) zu erhalten, 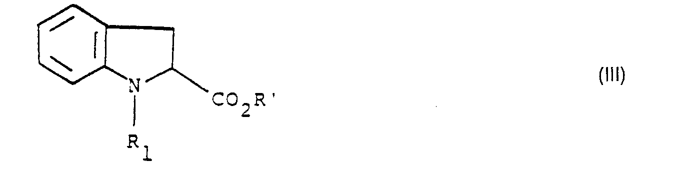 die man eventuel mit einem halogenid R₂Y in Gegenwart von Butyllithium und Diisopropylamin alkyliert oder allyliert um die Verbindung (IV) zu erhalten, 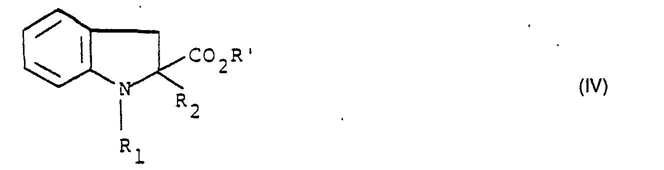 und man die Verbindung (lll) oder (IV) mit Äthylendiamin in Gegenwart von Trimethylaluminium in die Verbindung (I) umwandelt, wobei X und Y labile Gruppen sind, zum Beispeil lod oder Bromatome, R' ein (C₁₋₄)Alkylrest ist, und R, und R₂ die in Anspruch 1 gegebenen Bedeutungen haben.

4. Verfahren zur Herstellung der Verbindungen nach Anspruch 1, in welcher R₁ H ist, dadurch gekennzeichnet, dass man
entweder eine Verbindung (IV), in welcher R₁ die Benzylgruppe ist, debenzyliert und die erhaltene Verbindung (IV), in welcher R₁ H ist, in eine Verbindung (I), in welcher R, H ist, umwandelt, durch Reaktion der Verbindung (IV) mit Äthylendiamin in Gegenwart von Trimethylaluminium,
oder eine Verbindung (1), in welcher R, eine Benzylgruppe ist, debenzyliert.

5. Arzneimittel, dadurch gekennzeichnet, dass es eine Verbindung nach einem der Ansprüche 1 und 2 enthält.

6. Pharmazeutische Zubereitung, dadurch gekennzeichnet, dass sie eine Verbindung nach einem der Ansprüche 1 und 2 in Kombination mit irgendeinem geeigneten Hilfsstoff enthält.

## Patentansprüche (Patentansprüche für folgende(n) Vertragsstaat(en) : AT)

Verfahren zur Herstellung von Indol-Derivaten, in Form ihrer Racemate oder Enantiomeren, der Formel (I) 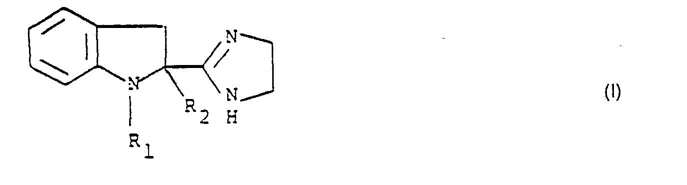 in welcher
R₁ ein Wasserstoffatom, einen geradkettigen oder verzweigten (C₁₋₆)Alkyirest, einen (C₃₋₆)Cycloalkyl-(C₁₋₄)Alkyest, den Allylrest, den Naphtylmethylrest, den Phenethylrest oder einen Benzylrest, der ein oder wehrere Halogenatome, Methylgruppen, Methoxygruppen oder Methylenedioxygruppen tragen kann, bedeutet,
dadurch gekennzeichnet, dass man
entweder die Verbindungen worin R₁ kein Wasserstoffatom ist, herstellt, wobei man ein Ester der Formel (ll) 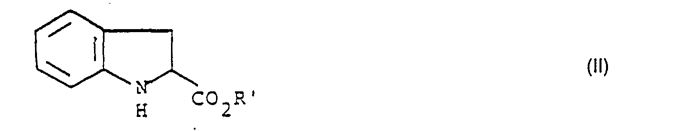 mit einer Verbindung R₁X reagiert, um die Verbindung (lll) zu erhalten, 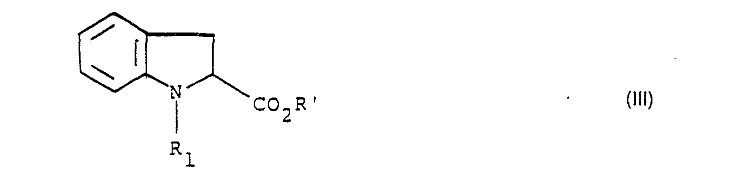 die man eventuel mit einem Halogenid R₂Y in Gegenwart von Butyllithium und Diisopropylamin alkyliert oder allyliert um die Verbindung (IV) zu erhalten, 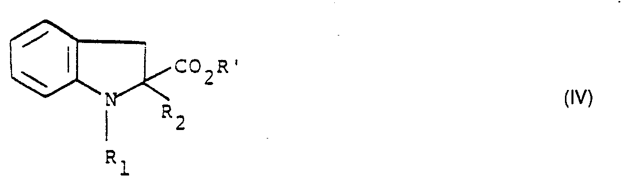 und man die Verbindung (lll) oder (IV) mit Äthylendiamin in Gegenwart von Trimethylaluminium in die Verbindung (I) umwandelt, wobei X und Y labile Gruppen sind, zum Beispiel lod oder Bromatome. R' ein (C₁₋₄)Alkylrest ist, und R, und R₂ die oben gegebenen Bedeutungen haben,
oder die Verbindungen worin R₁ H ist, herstellt, wobei man
entweder eine Verbindung (IV), in welcher R₁ die Benzylgruppe ist, debenzyliert und die erhaltene Verbindung (IV), in welcher R₁ H ist, in eine Verbindung (I), in welcher R₁ H ist, umwandelt. durch Reaktion der Verbindung (IV) mit Äthylendiamin in Gegenwart von Trimethylaluminium,
oder eine Verbindung (I), in welcher R₁ eine Benzylgruppe ist, debenzyliert.
